# EUROPEAN PATENT SPECIFICATION

(11) **EP 1 140 786 B1**
(45) Date of publication and mention of the grant of the patent: **30.11.2005**
(21) Application number: 99967437.7
(22) Date of filing: 20.12.1999
(51) Int. Cl.: C07C 209/28, C07D 243/14, C07D 233/34, C07D 333/64

(54) **REDUCTIVE ALKYLATION OF SECONDARY AMINES WITH HYDROSILANE**
REDUKTIVE ALKYLIERUNG SEKUNDÄRER AMINE MIT HYDROSILAN
ALKYLATION REDUCTRICE D'AMINES SECONDAIRES AU MOYEN D'HYDROSILANE

(30) Priority: 12.01.1999 US 115587 P
(43) Date of publication of application: 10.10.2001
(73) Proprietor: Bristol-Myers Squibb Company, Princeton, NJ 08543-4000 (US)
(72) Inventor: CHEN, Bang-Chi, Plainsboro, NJ 08536 (US); SUNDEEN, Joseph, E., Yardley, PA 19067 (US); GUO, Peng, Lawrenceville, NJ 08648 (US)
(74) Representative: Vossius & Partner
(86) International application number: PCT/US1999/030299
(87) International publication number: WO 2000/041992

(56) References cited:
- TESSA BEULSHAUSEN: "Asymmetric synthesis of diastereomerically and enantiomerically pure, 3-substituted (2S,2R)-N-methylserine esters" LIEBIGS ANNALEN DER CHEMIE, no. 5, 1992, pages 523-526, XP001068927
- JERRY MARCH: "Advanced Organic Chemistry" 1985 , JOHN WILEY & SONS XP002196521 * page 798 - page 799 *
- KURSANOV D.N. ET AL.: 'Applications of Ionic Hydrogenation to Organic Synthesis' SYNTHESIS, September 1974, pages 633 - 651, XP002923118
- DUBE D. ET AL.: 'Reductive N-Alkylation of Amides, Carbamates and Ureas' TET. LET., vol. 40, 1999, pages 2295 - 2298, XP002927259

## Description

The present invention concerns a new process for the reductive alkylation of secondary amines using a hydrotrialkylsilane with applications to the preparation of imidazole-containing benzodiazepines, inhibitors of famesyl protein transferase which find utility in the treatment of a variety of cancers and other diseases (Ding, C. Z.; Hunt, J. T.; Kim, S.-h.; Mitt, T.; Bhide, R.; Leftheris, K. WO 9730992; Chem. Abstr. 1998, 127, 278213).

Many methods have been previously reported for the reductive alkylation of secondary amines with carbonyl compounds in the presence of a reducing agent. The reducing agent includes: zinc (Lockemann, G. DE 503113; Chem. Abstr. 1931, 25, 522), H₂/Pd(or Pt, Ni) (Emerson, W. S. Org. React. 1948, 4, 174; Schaus, J. M.; Huser, D. L; Titus, R. D. Synth. Commun. 1990, 20, 3553), selenophenol (Fujimori, K.; Yoshimoto, H.; Oae, S. Tetrahedron Lett. 1980, 21, 3385), NaBH₄ (Verardo, G.; Giumanini, A. G.; Strazzolini, P. Synth. Commun. 1994, 24, 609), and NaCNBH₃ (Lee, M.; Garbiras, B. J. Synth. Commun. 1994, 24, 3129). All these reducing agents suffer one or more drawbacks. They are either toxic such as selenophenol and NaCNBH₃, or are not selective such as zinc, H₂/Pd(or Pt, Ni) and NaBH₄ since a number of other reducible groups could be affected by these reagents. NaBH(OAc)₃ was subsequently introduced to address these problems (Abdel-Magid, A. F.; Carson, K. G.; Harris, B. D.; Maryanoff, C. A.; Shah, R. D. J. Org. Chem. 1996, 61, 3849) and this reagent was used in the preparation of the aforementioned imidazole-containing benzodiazepine compounds (Ding, C. Z.; Hunt, J. T.; Kim, S.-h.; Mitt, T.; Bhide, R.; Leftheris, K., WO 9730992; Chem. Abstr. 1998, 127; 278213). Although the desired products were obtained from 1H-benzodiazepine starting materials, a large excess of aldehyde was required for a satisfactory conversion. In addition, expensive chromatographic separation of products was necessary which rendered this method not amenable to large scale preparation.

Reduction of imines preformed from primary anilines and aldehydes to secondary anilines with Et₃SiH/TFA has been previously reported (Kursanov, D. N.; Parnes, Z. N.; Loim, N. M. Synthesis 1974, 633; Loim, N. M. Bull. Acad. Sci. USSR, Div. Chem. Sci. 1968, 1345). Reduction of the preformed aminal from secondary amine and formaldehyde to give tertiary amine using Et₃SiH/TFA has also been disclosed (Beulshausen, T.; Groth, U.; Schoellkopf, U. Liebigs Ann. Chem. 1992, 523). However, it remains as a question whether the direct reductive alkylation of a secondary amine with an aldehyde using Et₃SiH/TFA would work, since it was well documented that aldehydes react readily with Et₃SiH/TFA to give the corresponding alcohols and even methylenes (Kursanov, D. N.; Pames, Z. N.; Loim, N. M. Synthesis 1974, 633; West, C. T.; Donnelly, S. J.; Kooistra, D.A.; Doyle, M. P. J. Org. Chem. 1973, 38, 2675).

This invention is a new efficient process for the preparation of tertiary amines by reaction of a secondary amine with an aldehyde or ketone compound in the presence of a hydrotrialkylsilane and a Lewis acid which is selected from trifluoroacetic acid, trifluoromethanesulfonic acid and boron trifluoride, with applications to the preparation of imidazole-containing benzodiazepines, inhibitors of famesyl protein transferase. The process involves a new reaction for the reductive alkylation of a secondary amine with an aldehyde using Et₃SiH/TFA. Compared with the previously reported synthesis, the new reaction of the present invention is faster, uses lesser amounts of aldehyde and gives a higher yield of the desired product.

Further embodiments of the present application are described in the dependent claims.

In its broadest aspect, the present invention provides a more efficient process for the preparation of tertiary amines (IV) from secondary amines (I) and carbonyl compounds (II), i.e., an aldehyde or keton. The process involves reaction of a secondary amine (I) with a carbonyl compound (II) using a hydrotrialkylsilane (III) in the presence of a Lewis acid selected from trifluoroacetic acid, trifluoromethanesulfonic, acid and boron trifluoride (Scheme 1).

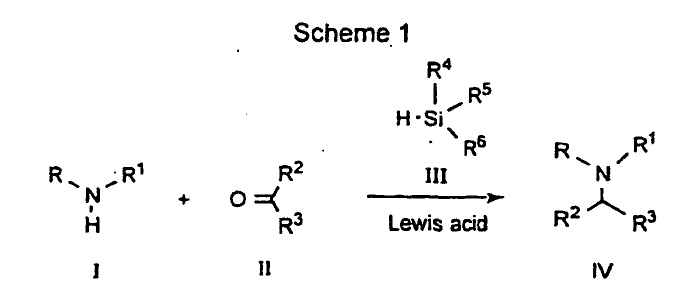

In a more narrow aspect, the invention deals with the preparation of 1-alkyl-2,3,4,5-tetrahydro-1,4-benzodiazepines (VII) from 4-substituted-2,3,4,5-tetrahydro-1,4-benzodiazepines (V) with an aldehyde (VI) using hydrosilane (III) in the presence of a Lewis acid as defined above (Scheme 2).

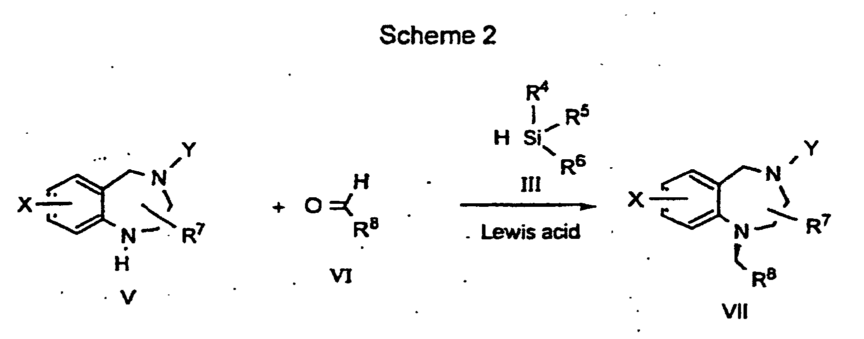

In compounds I, II, III, IV, V, VI and VII, the funtional groups R, R¹, R² R³, R⁴, R⁵, R⁶, R⁷ and R⁸ can be C₁-C₁₂ alkyl or aryl groups containing up to 30 carbon atoms. X can be one or a combination of hydrogen, halogen, R, RO-, RCOO-, ROCOO-, R¹R²NCOO-, R¹R²N-, RS-, RS(O)₂-, ROS(O)₂-, R¹,R²NS(O)₂-, CN, NO₂- Y can be RC(O)-, ROC(O)-, R₁R₂NC(O)-, RS(O)₂-, ROS(O)₂-, R₁R₂NS(O)₂-.

Unless otherwise indicated, as used herein the term "alkyl" or derivative forms thereof, refers to straight chain or branched alkyl groups of 1 to 12 carbon, atoms. Example of alkyl groups include methyl, ethyl, propyl, butyl, isobutyl, pentyl, hexyl and the like. The term "aryl" or derivative forms thereof refers to aryl groups of up to 30 carbon atoms. Examples of aryl groups include phenyl, naphthyl, anthryl, biphenyl and the like. The term "halo" refers to Cl, Br, and I.

Lewis acid includes protonic acids and non-protonic acids. Protonic acids include trifluoroacetic acid, trifluoromethanesulfonic acid. Non-protonic acids include boron trifluoride.

As set forth in Scheme 2, the process for the preparation of 1-alkyl-2,3,4,5-tetrahydro-1,4-benzodiazepines involves reaction of a 4-substituted-2,3,4,5-tetrahyrdro-1,4-benzodiazepine with an aldehyde in the presence of a hydrosilane and Lewis acid in a suitable solvent or solvent mixtures.

The 4-substituted-2,3,4,5-tetrahydro-1,4-benzodiazepine includes 4-acyl and 4-sulfonyl-2,3,4,5-tetrahydro-1,4-benzodiazepines with the latter preferred. The aldehyde includes aliphatic and aromatic aldehydes with imidazole-4-carbaxaldehyde being preferred. Of the hydrotrialkylsilane used in the reaction, hydrotriethylsilane is preferred. The Lewis acid used in the reaction includes trifluoroacetic acid, trifluoromethanesulfonic acid and boron trifluoride, with trifluoroacetic acid being the preferred Lewis acid. Suitable solvent(s) include hydrocarbons, halogenated hydrocarbons, ethers, esters, amides and nitriles. The preferred solvent is dichloromethane. The reaction temperatures range from -110 to 150°C with 0-100°C preferred.

### EXAMPLE 1 (Reference Example)

### 1H-3-(4-fluorobenzyl)-4-(phenylsulfonyl)-7-cyano-2,3,4,5-tetrahydro-1,4-benzodiazepine:

To a stirred solution of (3R)-3-(4-fluorobenzyl)-7-cyano-2,3,4,5-tetrahydro-1,4-benzodiazepine (3.6g) in anhydrous methylene chloride (50 mL) and diisopropylethylamine (3.5mL) was added phenylsulfonyl chloride (1.5mL) at 0°C in 20 minutes. The mixture was stirred at room temperature for 5 hours. Methylene chloride (50mL) and saturated sodium bicarbonate was added and the mixture was stirred for 1 hour. The phases were separated and the organic layer was washed with 1 N NaOH (2x20mL), 10% KHSO4 (2x20mL) and saturated brine. The solvent was removed to give an oil which was triturated with ethyl acetate to give 1H-3-(4-fluorobenzyl)-4-(phenylsulfonyl)-7-cyano-2,3,4,5-tetrahydro-1,4-benzodiazepine, 4.5g, 83%.

### EXAMPLE 2 (Reference Example)

### 1H-3-Benzyl-4-(2-thienylsulfonyl)-7-cyano-2,3,4,5-tetrahydro-1,4-benzodiazepine:

To a stirred solution of 3-benzyl-7-cyano-2,3,4,5-tetrahydro-1,4-benzodiazepine (34.5g) in anhydrous methylene chloride (500 mL) and diisopropylethylamine (45mL) was added a solution of 2-thienylsulfonyl chloride (34g) in methylene chloride (200 mL) at 0°C over 30 minutes. The mixture was stirred at room temperature for 40 hours. The solvent was removed to give an oil which was crystallized from ethanol (100mL) to give 1 H-3-benzyl-4-(2-thienylsutfonyl)-7-cyano-2,3,4,5-tetrahydro-1,4-benzodiazepine, 41 g, 76%.

### EXAMPLE 3

### 1-(Imidazole-4-yl)methyl)-3-(4-fluorobenzyl)-4-phenylsulfonyl-7-cyano-2,3,4,5-tetrahydro-1,4-benzodiazepine:

To a stirred solution of 1H-3-(4-fluorobenzyl-4-(phenylsulfonyl)-7-cyano-2,3,4,5-tetrahydro-1,4-benzodiazepine (3.6g) and imidazole-4-carboxaldehyde (1.7g) in anhydrous methylene chloride (40 mL) was added trifluoroacetic acid (30 mL). The mixture was stirred at room temperature for 30 minutes and hydrotriethylsilane (2.76 mL) was added. The reaction mixture was stirred at room temperature for 5 hours. The solvent was removed and the residue was dissolved in methylene chloride (100 mL) and stirred with saturated sodium bicarbonate. The phases were separated. The organic layer was washed with 2N sodium hydroxide (2x50mL). After drying, the solvent was removed to give a solid which was recrystallized from methanol (10mL) to give 1-(imidazole-4-yl)methyl)-3-(4-fluorobenzyl)-4-(phenylsulfonyl)-7-cyano-2,3,4,5-tetrahydro-1,4-benzodiazepine, 4.00g, 93%, MS, 502 (M+H).

### EXAMPLE 4

### 1-(Imidazole-4-yl)methyl)-3-benzyl-4-(2-thienylsulfonyl)-7-cyano-2,3,4,5-tetrahydro-1,4-benzodiazepine hydrochloride:

To a stirred solution of 1H-3-benzyl-4-(2-thienylsulfonyl)-7-cyano-2,3,4,5-tetrahydro-1,4-benzodiazepine (31.7g) and imidazole-4-carboxaldehyde (20.1g) in anhydrous methylene chloride (350 mL) was added trifluoroacetic acid (200 mL). The mixture was stirred at room temperature for 40 minutes. Hydrotriethylsilane (25 mL) was added to the reaction mixture over 15 minutes. After addition, the reaction mixture was stirred at room temperature overnight. The solvent was removed under vacuo. The residue was dissolved with methylene chloride (400 mL) and stirred with saturated sodium bicarbonate (100 mL) for 1 hour. The phases were separated. The organic layer was washed with 10% sodium hydroxide (3x100mL) and saturated brine (150mL) and dried. The solvent was removed to give a solid which was dissolved in ethyl acetate (200 mL). HCl in ether (1N, 150 mL) was added. The resulted slurry was filtered. The cake was washed with ethyl acetate and ether and dried to give 1-(imidazole-4-yl)methyl)-3-benzyl-4-(2-thienylsulfonyl)-7-cyano-2,3,4,5-tetrahydro-1,4-benzodiazepine hydrochloride, 40.87g, 95%, MS, 490 (M+H).

## Claims

1. A process for preparing a tertiary amine by reaction of a secondary amine with an aldehyde or ketone compound in the presence of a hydrotrialkylsilane and a Lewis acid which is selected from trifluoroacetic acid, trifluoromethanesulfonic acid and boron trifluoride.

2. The process of claim 1, wherein the secondary amine is 4-substituted-2,3,4,5-tetrahydro-1,4-benzodiazepine.

3. The process of claim 2, wherein the 4-substituted-2,3,4,5-tetrahydro-1,4-benzodiazepine is 3-benzyl-4-(2-thienylsulfonyl)-7-cyano-2,3,4,5-tetrahydro-1,4-benzodiazepine.

4. The process according to any of claims 1 to 3, wherein the aldehyde or ketone compound is an aldehyde.

5. The process of claim 4, wherein the aldehyde is imidazole-4-carboxaldehyde. substituted-2,3,4,5-tetrahydro-1,4-benzodiazepine.

6. The process according to any of claims 1 to 5, wherein the hydrotrialkylsilane is hydrotriethylsilane.

7. The process according to any of claims 1 to 6, wherein the Lewis acid is trifluoroacetic acid.

8. The process according to any of claims 1 to 7, wherein said tertiary amine is 1-alkyl-2,3,4,5-tetrahydro-1,4-benzodiazepine.

## Patentansprüche

1. Verfahren zur Herstellung eines tertiären Amins durch Umsetzen eines sekundären Amins mit einer Aldehyd- oder Ketonverbindung in Gegenwart eines Hydrotrialkylsilans und einer Lewis-Säure, welche aus Trifluoressigsäure, Trifluormethansulfonsäure und Bortrifluorid ausgewählt ist.

2. Verfahren nach Anspruch 1, wobei das sekundäre Amin 4-substituiertes 2,3,4,5-Tetrahydro-1,4-benzodiazepin ist.

3. Verfahren nach Anspruch 2, wobei das 4-substituierte 2,3,4,5-Tetrahydro-1,4-benzodiazepin 3-Benzyl-4-(2-thienylsulfonyl)-7-cyano-2,3,4,5-tetrahydro-1,4-benzodiazepin ist.

4. Verfahren nach einem der Ansprüche 1 bis 3, wobei die Aldehyd- oder Ketonverbindung ein Aldehyd ist.

5. Verfahren nach Anspruch 4, wobei der Aldehyd Imidazol-4-carboxaldehyd ist.

6. Verfahren nach einem der Ansprüche 1 bis 5, wobei das Hydrotrialkylsilan Hydrotriethylsilan ist.

7. Verfahren nach einem der Ansprüche 1 bis 6, wobei die Lewis-Säure Trifluoressigsäure ist.

8. Verfahren nach einem der Ansprüche 1 bis 7, wobei das tertiäre Amin 1-Alkyl-2,3,4,5-tetrahydro-1,4-benzodiazepin ist.

## Revendications

1. Un procédé de préparation d'une amine tertiaire par réaction d'une amine secondaire avec un composé d'aldéhyde ou de cétone en présence d'un hydrotrialkylsilane et d'un acide de Lewis qui est sélectionné parmi l'acide trifluoroacétique, l'acide trifluorométhanesulfonique et le trifluorure de bore.

2. Le procédé de la revendication 1, dans lequel l'amine secondaire est le 2,3,4,5-tétrahydro-1,4-benzodiazépine substitué en 4.

3. Le procédé de la revendication 2, dans lequel le 2,3,4,5-tétrahydro-1,4-benzodiazépine substitué en 4 est du 3-benzyl-4-(2-thiénylsulfonyl)-7-cyano-2,3,4,5-tétrahydro-2,4-benzodiazépine.

4. Le procédé selon l'une quelconque des revendications 1 à 3, dans lequel le composé d'aldéhyde ou de cétone est une aldéhyde.

5. Le procédé de la revendication 4, dans lequel l'aldéhyde est l'imidazole-4-carboxaldéhyde.

6. Le procédé selon l'une quelconque des revendications 1 à 5, dans lequel l'hydrotrialkylsilane est l'hydrotriéthylsilane.

7. Le procédé selon l'une quelconque des revendications 1 à 6, dans lequel l'acide de Lewis est l'acide trifluoroacétique.

8. Le procédé selon l'une quelconque des revendications 1 à 7, dans lequel l'amine tertiaire est la 1-alkyl-2,3,4,5-tétrahydro-1,4-benzodiazépine.
